# EUROPEAN PATENT SPECIFICATION

(11) **EP 4 294 332 B1**
(45) Date of publication and mention of the grant of the patent: **14.10.2026**
(21) Application number: 22757079.3
(22) Date of filing: 22.02.2022
(51) Int. Cl.: A61F 2/78, A61F 2/50

(54) **KNIT PROSTHETIC LINER TEXTILE WITH DIFFERENTIATED KNIT FABRIC EXTERIOR INCORPORATING LOW EXTENSIBILITY STRIPS**
GESTRICKTE PROTHESENEINLAGE MIT DIFFERENZIERTER STRICKGEWEBEAUSSENSEITE MIT STREIFEN MIT GERINGER DEHNBARKEIT
TEXTILE DE REVÊTEMENT DE PROTHÈSE TRICOTÉ AYANT UN EXTÉRIEUR DE TISSU TRICOTÉ DIFFÉRENCIÉ INCORPORANT DES BANDES À FAIBLE EXTENSIBILITÉ

(30) Priority: 22.02.2021 US 202117181110
(43) Date of publication of application: 27.12.2023
(73) Proprietor: Alps South Europe s.r.o, 32600 Plzen (CZ); Laghi, Aldo, Pinellas Park, FL 33782 (US)
(72) Inventor: VINT, Nathaniel, Palm Harbor, FL 34684 (US); LAGHI, Aldo, Pinellas Park, FL 33782 (US)
(74) Representative: Franks & Co Limited
(86) International application number: PCT/US2022/017184
(87) International publication number: WO 2022/178368

(56) References cited:
- US-A1- 2013 331 951
- US-A1- 2019 070 024
- US-A1- 2019 070 024
- US-A1- 2021 154 030
- US-A1- 2021 186 719
- US-B1- 6 231 617
- US-B1- 6 964 688
- US-B2- 8 597 368

## Description

### CROSS-REFERENCE TO RELATED APPLICATIONS

This application claims priority to pending Application No. 17/181,110, titled "Knit Prosthetic Liner Textile With Differentiated Knit Fabric Exterior Incorporating Low Extensibility Strips," filed February 22, 2021, which is a continuation-in-part of Application No. 16/120,929, titled "Knit Prosthetic Liner Textile with Differentiated Knit Fabric Exterior," filed September 4, 2018, which claims priority to U.S. Provisional Patent Application No. 62/554,102, filed September 5, 2017. This application is also a continuation-in-part of Application No. 17/094,885 titled "Low Extensibility Strips for Prosthetic and Orthotic Application," filed November 11, 2020, which claims priority to U.S. Provisional Patent Application No. 62/942,388 filed December 2, 2019 and to U.S. Provisional Patent Application No. 62/938,483 filed November 21, 2019.

### BACKGROUND OF THE INVENTION

### Field of the Invention

This invention relates to liners for use in a prosthetic assembly. More particularly, the described invention relates to liners having a particular stitching such that the distal end of the liner has less stretch than the proximal end of the liner so as to reduce pistoning which incorporate low extensibility strips which reduce elongation in the longitudinal direction when applied to prosthetics and orthotics.

### Description of the Background Art

Prosthetic liners have been in use since the 1970's, mostly custom made, and made of various materials. Silicone liners have been used since the 1980s in the prosthetic industry such as those described in U.S. Pat. No. 4,923,474 granted to Klasson and Kristinsson. Other examples of such liners include U.S. Pat. No. 5,728,168 to Laghi et al., U.S. Pat. No. 5,830,237 granted to Kania, U.S. Patent No. 5,507,834 to Laghi et al., U.S. Patent No. 5,443,525 to Laghi et al., and U.S. Patent No. 5,728,168 to Laghi et al. Gel and urethane liners have also been used for prosthetic and orthotic purposes and, for the most part, have a fabric covering. The fabric covering is used to reinforce the underlying material (silicone, gel, urethane) and allows for extensibility to make it easier to don and doff the liner by rolling it on and off the residual limb

The liners disclosed in U.S. Patent No. 5,443,525, U.S. Patent No. 5,507,234, U.S. Patent No. 5,728,168, U.S. Patent No. 6,544,292, and U.S. Patent No. 6,764,631 each represent advances in the field of fabric covered liners. Additionally, U.S. Patent No. 6,454,812 describes a liner comprising additional features attached to the liner textile to limit the vertical stretch at the distal end of a liner. This method has been used successfully within the field of prosthetics to provide the liner textile with a strengthened distal end, means for attaching a threaded mechanical feature, and a boundary layer that prevents the thermoplastic elastomer from undesirably passing through the textile during high pressure processing.

Fabric liners having a lower longitudinal elongation at the distal end were initially popular because they prevented "pistoning." Pistoning is the loss of suspension of a residual limb when weight is taken off of it and the resulting pushing down of the limb on the socket when weight is applied, like the piston of car. This means that every time the amputee attempted to move his/her leg, there would be a delay between when the residual limb moved and when the prosthesis moved. Pistoning is more pronounced in locking liners, i.e. liners that attach the prosthesis to the residual limb by means of a distal umbrella and distal pin with a corresponding lock. Older versions of fabric liners with lower longitudinal elongation at the distal end prevent pistoning but also constrain the knee flexion of the amputee. If a liner exhibits a high longitudinal elongation throughout its length, then the prosthesis will move up and down during ambulation. On the other hand, if there is no longitudinal elongation then the amputee will find it difficult to flex the knee.

In order to overcome this issue, liners with a distal matrix were developed such as the liner described in U.S. Patent No. 6,454,812, described above. These liners include an additional fabric matrix which is knit to minimal longitudinal elongation where the matrix is bonded to the interior of the external fabric of the liner. However, this construction is expensive to construct due to the fact that the additional steps of applying adhesive to the interior of the exterior fabric, applying the reinforcing matrix to the adhesive layer, and curing of the adhesive are required. The present invention overcomes this problem by eliminating the reinforcing matrix and replaces it with a differentiated knitting of the exterior fabric.

There exists a need for liners with limited extensibility in localized regions of the residual limb and greater extensibility in other regions of the residual limb. Liners such as those described in U.S. Pat. No. 9,216,099 to Laghi, U.S. Pat. No. 9,364,347, U.S. Patent 8,394,150, U.S. Pat. No. 8,852,291, U.S. Pat. No. 8,246,694, U.S. Pat. No. 8,808,294, U.S. Pat. No. 8,226,732, U.S. Pat. No. 8,357,206, U.S. Pat. No. 6,764,631, U.S. Pat. No. 6,544,292, U.S. Pat. No. 6,454,812, U.S. Pat. No. 5,728,168 and U.S. Pat. No. 5,507,834 each describe liners which highlight such a need.

U.S. Patent No. 6,231,617 to Fay describes the a liner which incorporates elongate arms of a strip or ribbon shape. However, a limitation of Fay is that the arms have to radiate from the distal attachment plate or mounted to a peripheral edge of the distal attachment plate. The present invention allows for the use of low extensibility strips anywhere on or in the prosthetic/orthotic without being limited to a certain location on the device.

Generally, the distal end of locking liners require the fabric to be less extensible in the longitudinal direction or that a polymer or other solid implement be used in order to reduce pistoning of the prosthetic implement during ambulation or movement. Other regions may benefit from reduced extensibility of the fabric cover such as in the case the above the knee amputees who may exhibits unusually fleshy residual limbs.

The present invention utilizes stitching with reduced stretch regions such that the distal end of the liner has less stretch than the proximal end so as to prevent pistoning.

Therefore, it is an object of this invention to provide an improvement which overcomes the aforementioned inadequacies of the prior art devices and provides an improvement which is a significant contribution to the advancement of the liner art.

Another object of the invention is to provide a liner for prosthetic assemblies that reduces pistoning of the residual limb when in use.

Another object of the invention is to provide a liner for prosthetic assemblies that can also be used with a locking prosthetic assembly.

Another object of the invention is to provide a liner that implements two or more stitching types.

Another object of the invention is to provide a liner with less stretch capability at the distal end than at the proximal end.

Another object of the invention is to provide a liner with variable stretch characteristics based on knit construction and stitching.

Another object of the invention is to provide low extensibility material which, when applied to fabric liners, reduce extensibility in the longitudinal direction while maintaining extensibility in the transversal direction.

Another object of the invention is provide a fast and inexpensive way to reduce the extensibility of fabric liners in desired regions.

Another object of the invention is to provide a customizable solution for patient comfort such that a prosthetist can adhere appropriate shapes in appropriate locations in order to support or contain a region or segment of the residual limb as required by patient clinical conditions.

Another object of the invention is to contain the dynamic deformation of the residual limb during ambulation.

The foregoing has outlined some of the pertinent objects of the invention. These objects should be construed to be merely illustrative of some of the more prominent features and applications of the intended invention. Many other beneficial results can be attained by applying the disclosed invention in a different manner or modifying the invention within the scope of the disclosure. Accordingly, other objects and a fuller understanding of the invention may be had by referring to the summary of the invention and the detailed description of the preferred embodiment in addition to the scope of the invention defined by the claims taken in conjunction with the accompanying drawings.

### SUMMARY OF THE INVENTION

The present invention relates generally to a liner for use in a prosthetic assembly having variable stretch regions at a distal end providing different stretch regions incorporating low extensibility strips.

Specifically, the present invention relates to a prosthetic liner having a lower longitudinal stretch in the distal region than in either the proximal or optional intermediate region. The distal region may stretch anywhere from 0-30% vertically and 10-200% horizontally as compared to the proximal region's vertical stretch of 55-125% and 100-175% horizontally. The stretch of the liner at various pressure sensitive regions of a residual limb can also be lowered or heightened depending on the area. A variety of stitches may be used at the distal end to implement this lowered longitudinal stretch. Preferably, the liner is made of a stretchable material. The liner also has an interior layer of elastomer gel. The design of this prosthetic liner is primarily to prevent the "pistoning" of the amputee's residual limb within the liner and for comfort over pressure-sensitive areas of the residual limb. The liner also includes the use of a low extensibility material which is adhered to the fabric portion of a prosthetic liner or orthotic liner either internally or externally and limits the longitudinal movement while allowing for transversal movement. The customizable nature of the invention allows for shaping and personalization depending on the needs of the user.

The foregoing has outlined rather broadly the more pertinent and important features of the present invention in order that the detailed description of the invention that follows may be better understood so that the present contribution to the art can be more fully appreciated. Additional features of the invention will be described hereinafter which form the subject of the claims of the invention. It should be appreciated by those skilled in the art that the conception and the specific embodiment disclosed may be readily utilized as a basis for modifying or designing other structures for carrying out the same purposes of the present invention.

### BRIEF DESCRIPTION OF THE DRAWINGS

For a more complete understanding of the present disclosure and its advantages, reference is now made to the following descriptions, taken in conjunction with the accompanying drawings, in which:
Fig. 1 is a cross-sectional view of the present invention for use with a locking prosthetic assembly comprising two different types of stitches showing the different relevant regions;
FIG. 2 is a cross-sectional view of the present invention showing below-the-knee amputation pressure sensitive areas;
Fig. 3 is a cross-sectional view of the present invention showing below-the-knee-amputation pressure tolerant areas;
Fig. 4 is a cross-sectional view of the present invention showing above-the-knee amputation pressure sensitive areas;
Fig. 5 is a cross sectional view of the present invention showing above-the-knee amputation pressure tolerant areas;
Figs. 6A-6N are various stitching types that can be used in the distal region of the present invention to prevent pistoning;
Fig. 7 is a cross-sectional view of a liner incorporating the low extensibility material internally;
Fig. 8 is a top view of the low extensibility material in one preferred shape;
Fig. 9 is a top view of the low extensibility material in a second preferred shape;
Fig. 10 is a top view of the low extensibility material in a third preferred shape and configuration;
Fig. 11 is a top view of the low extensibility material in a second preferred configuration;
Fig. 12 is a cross-sectional view of a liner with an alternative arrangement of low extensibility material ;
Fig. 13 is a cross-sectional view with an alternative arrangement of low extensibility material;
Fig. 14 is a perspective view of a wrist orthotic with the low extensibility material adhered externally, which does not form part of the claimed invention;
Fig. 15 is a perspective view of a back orthotic with the low extensibility material adhered externally, which does not form part of the claimed invention;
Fig. 16A is a perspective view of an ankle orthotic with the low extensibility material adhered externally, which does not form part of the claimed invention;
Fig. 16B is a cross-sectional view of the ankle orthotic shown in Fig. 16A, which does not form part of the claimed invention;
Fig. 17 is a perspective view of a knee orthotic with the low extensibility material adhered externally, which does not form part of the claimed invention; and
Fig. 18 is a perspective view of an elbow orthotic with the low extensibility material adhered externally, which does not form part of the claimed invention.

Similar reference numerals refer to similar parts throughout the several views of the drawings.

### DETAILED DESCRIPTION OF THE PREFERRED EMBODIMENT

In this description one inch is regarded as 2,54cm. The following description is of the best mode presently contemplated for carrying out the invention. This description is not to be taken in a limiting sense, but is made merely for the purpose of describing one or more preferred embodiments of the invention. The scope of the invention should be determined with reference to the claims.

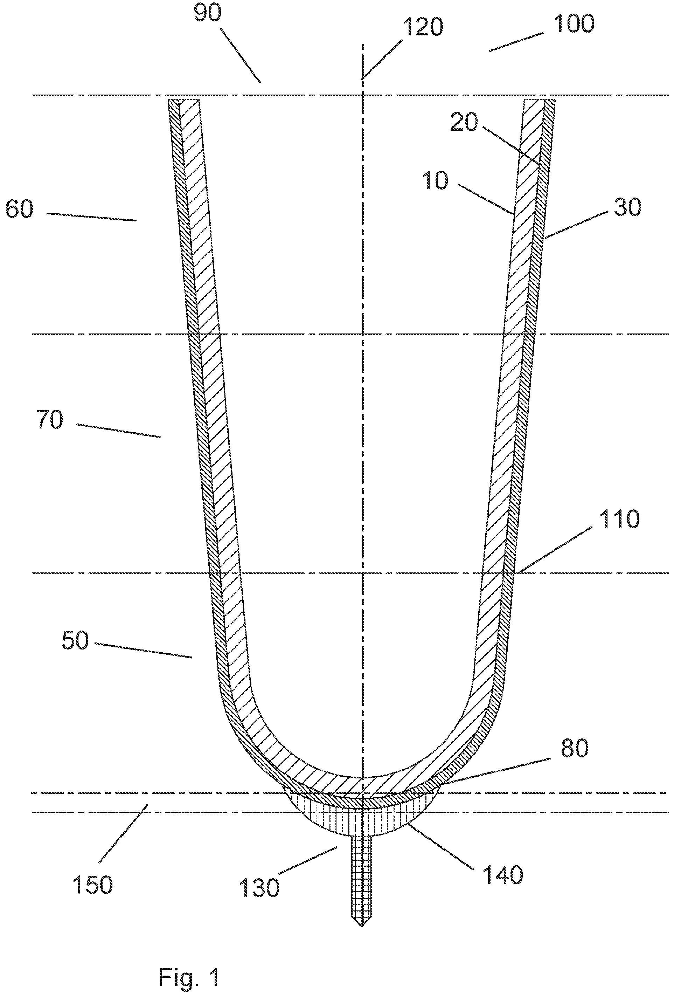

The present invention relates to a liner **100** for use with prosthetic devices. As shown in Fig. 1, the liner **100** for use with a prosthetic assembly comprises a plurality of types of stitching. The liner **100** comprises a distal region **50,** proximal region **60,** and an optional intermediate region **70.** In one embodiment, the distal region **50** that comprises the distal end **80** of the liner is constructed with a reduced vertical stretch when compared to the upper region **60** of the liner. Preferably, the distal region 80 has a vertical stretch of 0-30% and a horizontal stretch of 10-200% as compared to the proximal region **60,** which preferably has a vertical stretch of 55-125% and a horizontal stretch of 100-175%. Optionally, the intermediate region **70** may have a vertical stretch of 15-40% and a horizontal stretch of 80-120% or it may have the same stretch characteristics of the proximal region **60.** The reduced stretch of the distal region **50** is achieved on a flatbed knitting machine by use of a variety of different stitches as described below. In a preferred embodiment, the liner **100** is knit in a single piece construction with the proximal end **90** being open and the distal end **80** being closed. Alternatively, the two halves may be independently constructed and then sewn or attached together by another means to form the completed liner textile.

Preferably, the liner **100** is knit using computerized flatbed knitting machines that allow the use of several different yarns at the same time or sequentially in the same garment, including elastomeric fibers such as Lycra, latex, and silicone among others. These machines also allow the use of different stitch types in different areas of the same garment and controls the tension of each yarn being knitted. Further, an interior gel layer 10 resides on a limb face **22** of exterior fabric layer **30.** The gel layer **10** comprises a styrene-based polymer.

In another embodiment, the liner **100** comprises regions of differing horizontal and vertical stretch stitching depending on anatomical features related to the residual limb or mechanical features related to the prosthetic socket. This means that the boundary **110** between the distal region **50** and the proximal region **60** (or intermediate region **70** if present) may shift location. The boundary **110** is preferably generally perpendicular to the central axis **120** of the liner **100.** Additionally, the distal end **70** may house a locking mechanism **130.** The use of a locking mechanism **130** requires a construction that is strong enough for the distal attachment plate **140** to be retained by the fabric or else a catastrophic failure of the prosthesis may result. Typically, such locking mechanisms **130** are secured to the fabric through the use of a grommet that bites into the fabric or other similar means of attachment. In order for the fabric to withstand the localized stress imparted by the grommet, the construction of the knit at the distal end must be heavier and stronger than the rest of the fabric cover. Such construction cannot be used in the intermediate region **70** or proximal region **60** because it would impart poor functionality to the liner and limit the user's movement.

More specifically, the fabric in the locking mechanism region **150** must have little to no longitudinal elongation but high circumferential elongation in order to comfortably accommodate the residual limb. The locking mechanism region **150** is limited to the area juxtaposed to the locking mechanism **130.** The thickness of the fabric in the locking mechanism region **150** should greater than the thickness of the fabric in the distal region **50.** The distal region **50,** as compared to the locking mechanism region **150,** must have a greater degree of longitudinal elongation in order to facilitate knee flexion. The intermediate region 70 may have an even greater degree of longitudinal elongation and circumferential elongation than the distal region **50** to provide greater comfort to the amputee when sitting or moving.

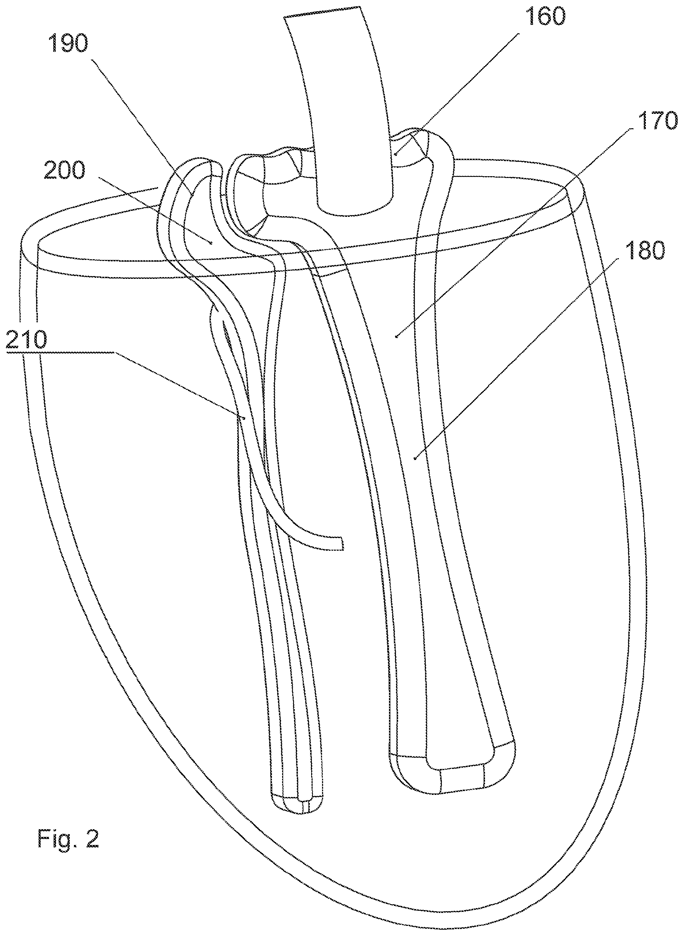

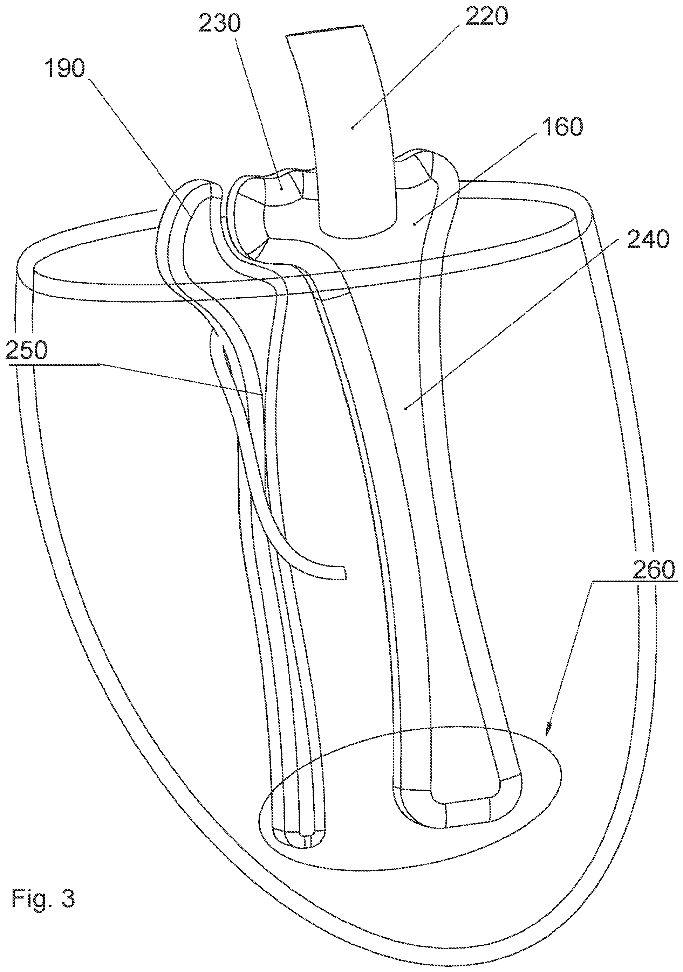

In another embodiment, the liner textile comprises regions of differing horizontal and vertical stretch depending on anatomical features related to the residual limb or mechanical features related to the prosthetic socket. There are various areas of the lower limbs that are more pressure sensitive, requiring more elongation, and various areas that are more pressure tolerant, which require less elongation. As shown in Fig. 2, the pressure sensitive areas for below-the- knee amputations comprise the portions of the tibia **160** such as the anterior tibia **170** and the anterior tibial crest **180** as well as parts of the fibula **190** such as the fibular head and neck **200,** and the fibular nerve **210.** These regions of the body exhibit sensitivity to pressure and therefore require a liner having greater longitudinal or circumferential elongation at those points. On the other hand, as shown in Fig. 3, the pressure-tolerant areas for below-the-knee amputations comprise the patellar tendon **220,** the medial tibia plateau **230,** the tibial shaft **240,** the fibular shaft **250,** and the distal end **260** of tibia **160** and fibula **190.** These regions may have more restricted longitudinal or circumferential elongation due to the tolerance these regions exhibit.

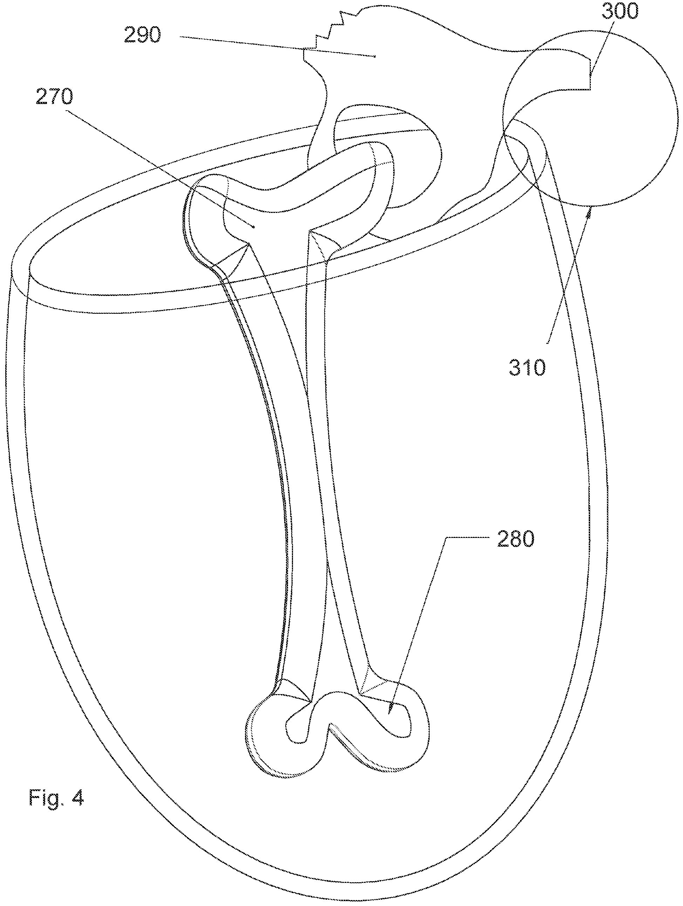

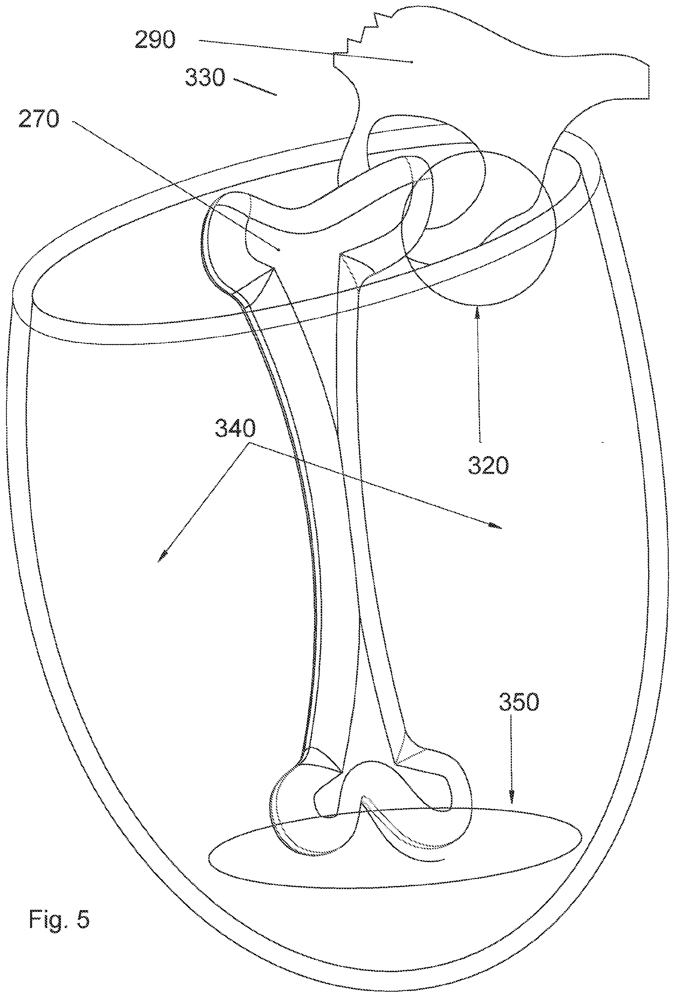

Similarly, as shown in Figs. 4 and 5, above-the-knee amputations have similarly pressure sensitive and pressure tolerant areas. The pressure sensitive areas comprise the distolateral end **280** of the femur **270,** the pubic symphysis **300** of the pelvic bone **290,** and the perineal area **310.** The pressure tolerant areas comprise the ischial tuberosity **320** of the pelvic bone **290,** the gluteals **330,** the lateral sides **340** of the residual limb, and the distal end **350** of the femur **270.** As with below-the-knee, pressure sensitive areas of above-the-knee amputations require greater longitudinal or circumferential elongation at those points while the pressure tolerant areas may have more restricted longitudinal or circumferential elongation due to the tolerance those regions exhibit.

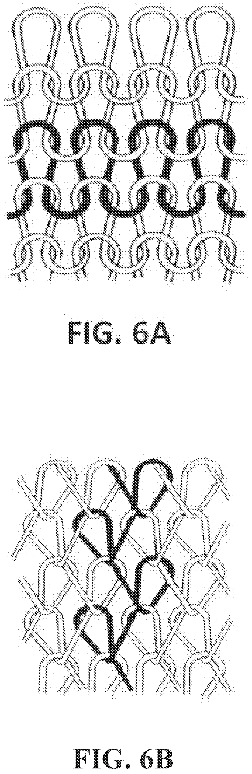

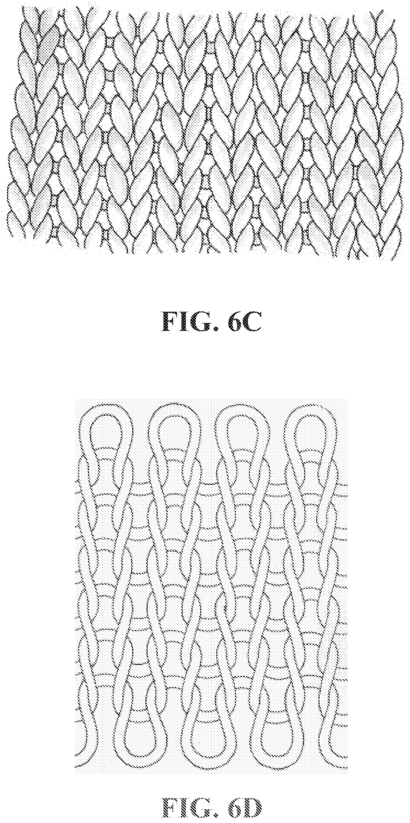

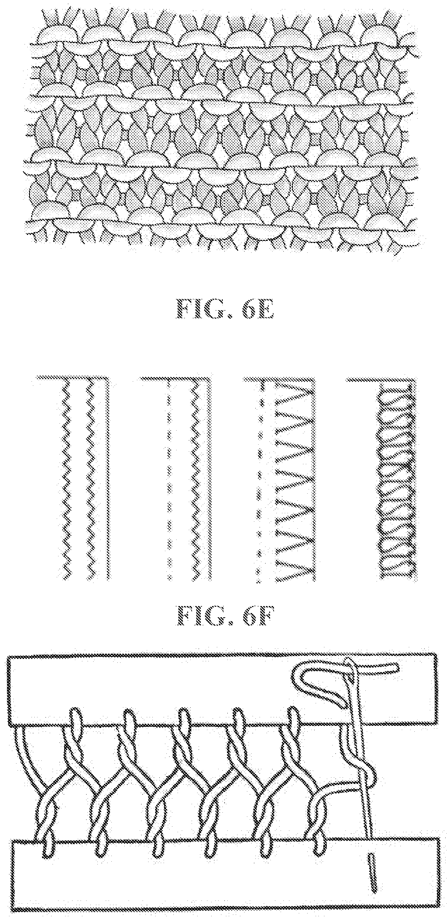

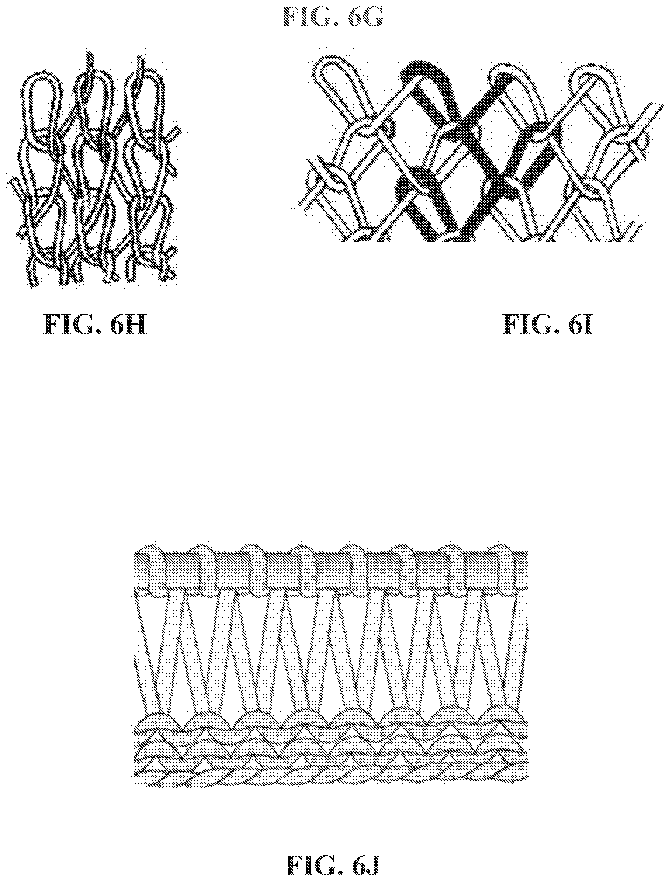

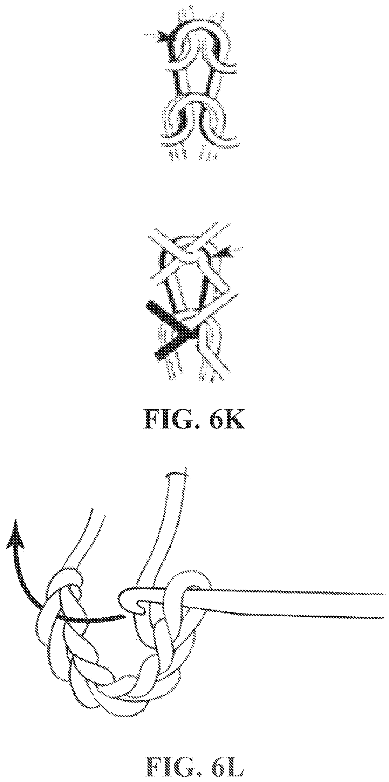

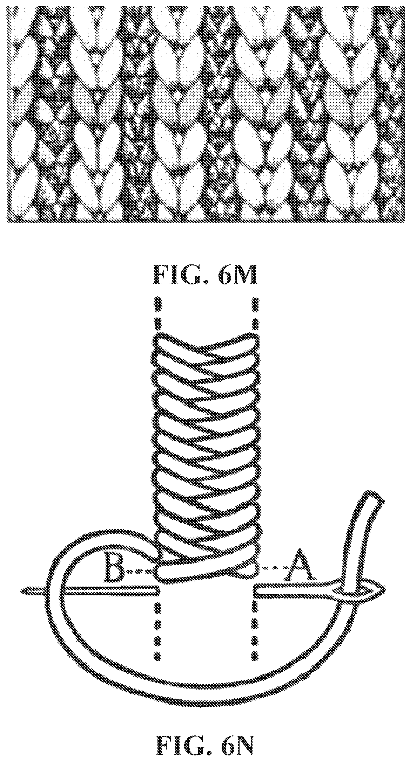

The fabric of the liner can be constructed in a number of ways so as to provide the required functionality. The functionality can be altered by using different yams or deniers, different elastomers, different weights of elastomers, and different stitches, among other ways, to provide the right longitudinal and circumferential elongation along the length of the liner. Typical yarn types include, but are not limited to, polyester, nylon, acrylic, cellulosic, aramid, natural fibers, and metal wires. Typical elastomers include Lycra/polyurethane, natural rubber, nitrile, and silicone. Typical stitch types, as shown in Figs. 6A-6N, include weft (Fig. 6A), warp (Fig. 6B), stockinette (Figs. 6C and 6D), garter (Fig. 6E), seam (Fig. 6F), fagoting (Fig. 6G), tricot (Figs. 6H and 6I), elongated (Fig. 6J), plated (Fig. 6K), slip (Fig. 6L), dip (Fig. 6M), and basket (Fig. 6N) and are used solely in the distal region **50** of the liner **100** so as to differentiate the elongation found in the proximal region **60.**

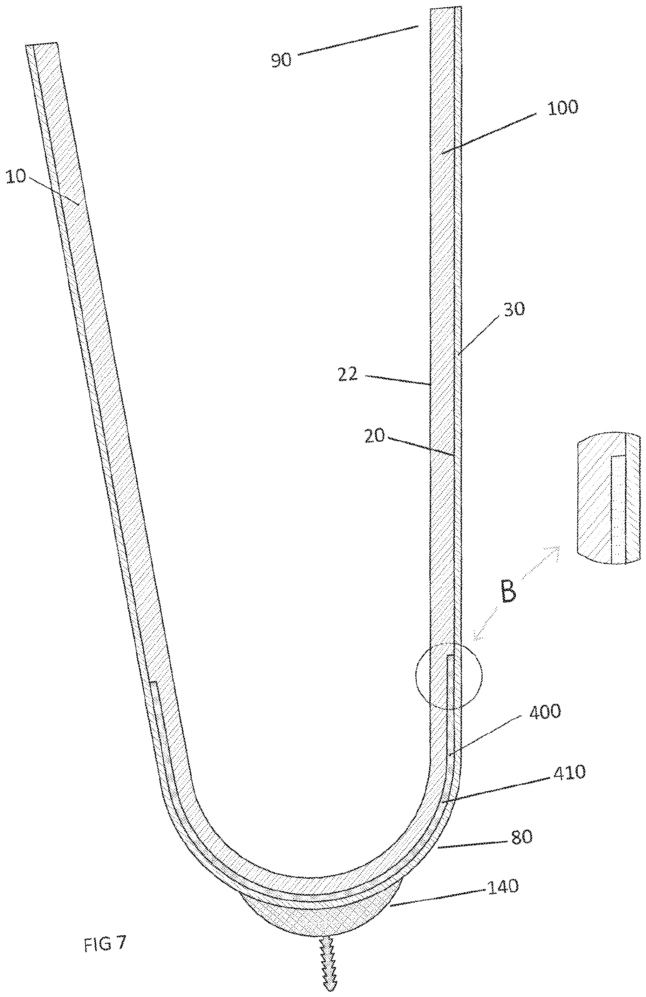

The present invention relates to a low extensibility material **400** for use with prosthetic or orthotic devices using the knit design described above. As shown in Fig. 7, this low extensibility material **400** can be used with a prosthetic liner **100** having a distal attachment plate **140** at a distal end **80.** The low extensibility material **400** for use with a prosthetic or orthotic assembly preferably comprises a strip of material **410** preferably made of strips of silk, fiberglass cloth, and other non-elastic materials like carbon fiber and thermoplastics. The prosthetic liner **100** comprises an elastomer layer **10** having a limb face **22** and a fabric face **20** wherein the fabric face **20** of the elastomer layer **10** is adhered to the external fabric **30** of the prosthetic liner **100.** In relation to the prosthetic liner 100, the low extensibility material **400** can be adhered to the fabric **30** either internally or not according to the present invention, externally. If internal placement is desired, the low extensibility material **400** is placed between the elastomer layer **10** and the fabric **30** in the desired region and adhered only to the fabric **30** as shown in the exploded view Part B. Preferably, the low extensibility material **400** is placed at the distal end **80** of the prosthetic liner **100** and extends towards the proximal end **90** of the prosthetic liner **100.** The low extensibility material **400** preferably extends upward towards the proximal end **90** of the prosthetic liner **100,** stopping at least an inch from the proximal end **90,** when placed at the distal end **80.** Similarly, the width of the low extensibility material **400** is between 0.5-4 inches such that it can be placed in a variety of locations and positions.

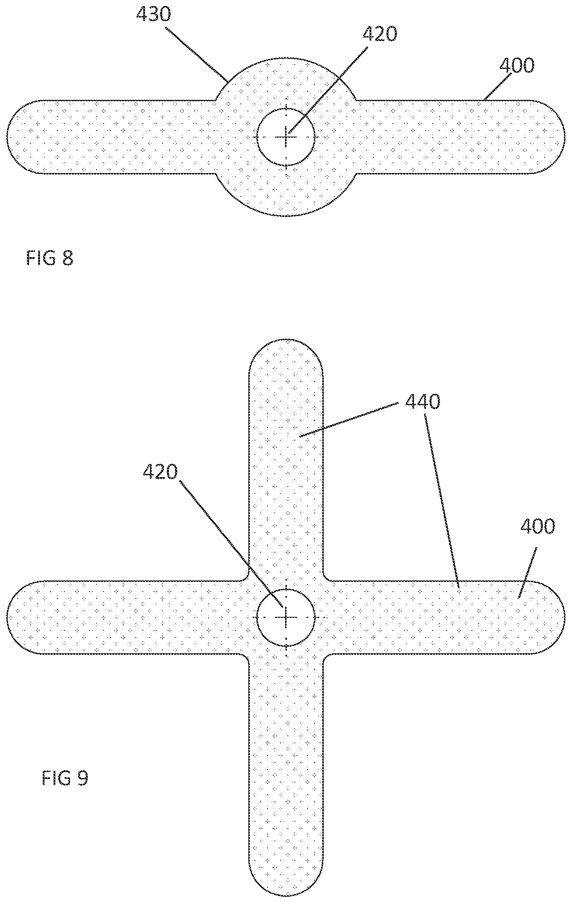

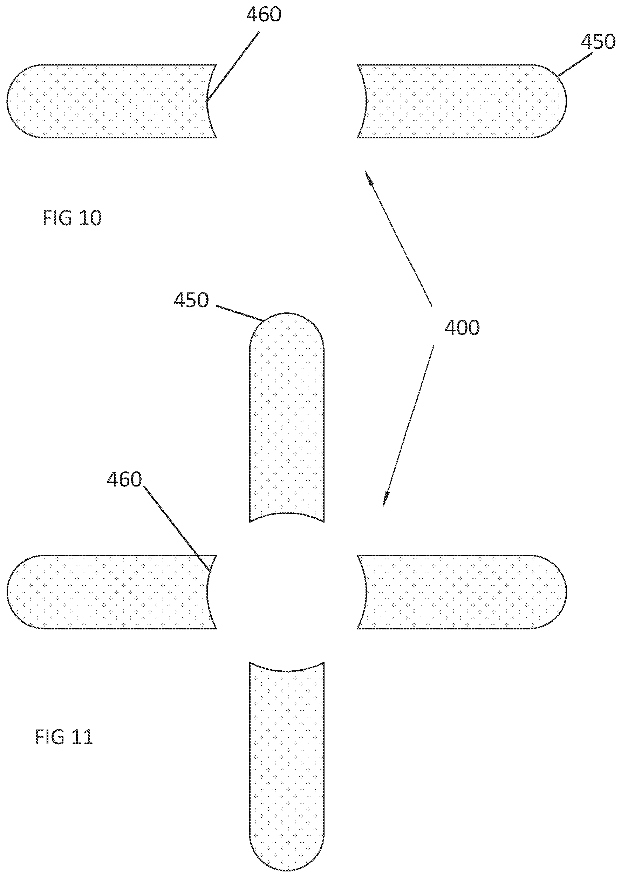

Figs. 8-13 show the low extensibility material **400** is a variety of configurations. Fig. 8 shows the low extensibility material **400** as a strip of material having a central aperture **420** with a bulged mid-region **430.** The locking mechanism **130,** as shown in Fig. 7, can be fed through the central aperture **420** if the low extensibility material **400** is adhered externally to the fabric **30.** As shown in Fig. 9, the low extensibility material **400** can also be configured to have multiple arms **440** extending radially outward from the central aperture **420.** As shown in Figs. 10 and 11, the central aperture **420** is optional. The low extensibility material **400** can be shaped such that a distal strip end **450** is convex while a proximal strip end **460** is concave. This allows for the low extensibility material **400** to be placed around the distal attachment plate **140** if desired or around the user's joint without impinging on joint movement.

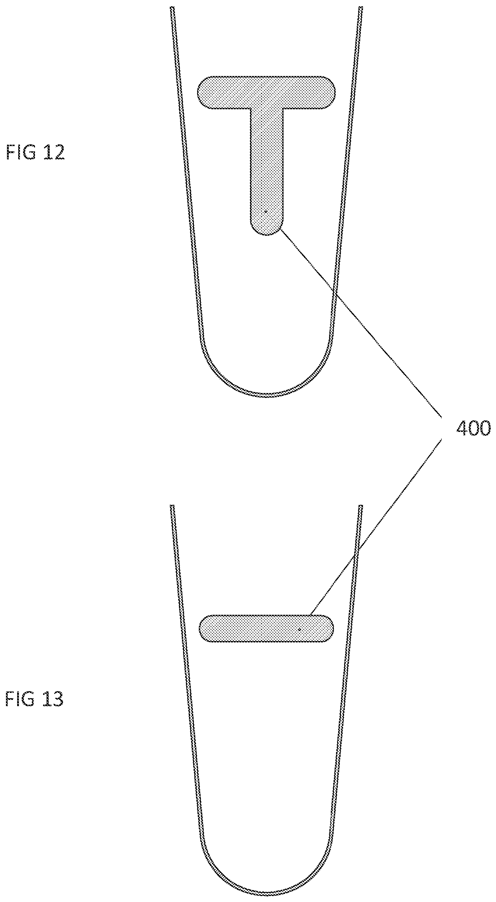

As seen in Figs. 12 and 13, the low extensibility material **400** can be placed in a variety of configurations according to the needs of the user. The T-shape and single strip configuration shown are not the only configurations that would be helpful to a user and any configuration determined by a medical professional to be medically helpful can be used.

Use of low extensibility material **400** as described herein has been tested and conclusively shows that use of the low extensibility material **400** limits the longitudinal stretch of the liner **100** while maintaining transversal stretch. In the first table, transversal stretch was tested:

| | Reinforced | | | | | Not Reinforced | | | |
|---|---|---|---|---|---|---|---|---|---|
| | before | after | change | % | | before | after | change | % |
| 1 | 13 | 21 | 8 | 61.53846 | 1 | 13 | 20.375 | 7.375 | 56.73077 |
| 2 | 13 | 22 | 9 | 69.23077 | 2 | 13 | 21.5 | 8.5 | 65.38462 |
| 3 | 13 | 20.125 | 7.125 | 54.80769 | 3 | 13 | 22 | 9 | 69.23077 |
| Average | | | | 61.85897 | Average | | | | 63.78205 |

As seen in the table above, "before" refers to the liner in a static state, meaning no weight was applied, and "after" refers to the liner at failure (i.e. until the strip tore). The number measured is circumference in inches. As can be seen from the test data, the transversal stretch was essentially the same whether the liner was reinforced with the low extensibility material 400 or not.

A similar test was performed testing the longitudinal stretch:

| | Reinforced | | | | | Not Reinforced | | | |
|---|---|---|---|---|---|---|---|---|---|
| | before | after | change | % | | before | after | change | % |
| 1 | 4.25 | 4.5 | 0.25 | 5.882353 | 1 | 4 | 4.75 | 0.75 | 18.75 |
| 2 | 4.25 | 4.5 | 0.25 | 5.882353 | 2 | 4.25 | 4.75 | 0.5 | 11.76471 |
| 3 | 4.5 | 4.75 | 0.25 | 5.555556 | 3 | 4.25 | 4.625 | 0.375 | 8.823529 |
| Average | | | | 5.77342 | Average | | | | 13.11275 |

As can be seen from this test, the longitudinal stretch was reduced by more than half when the low extensibility material **400** was used.

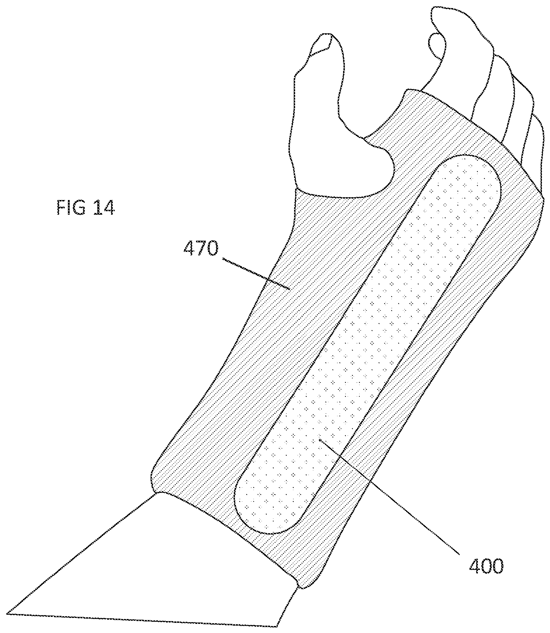

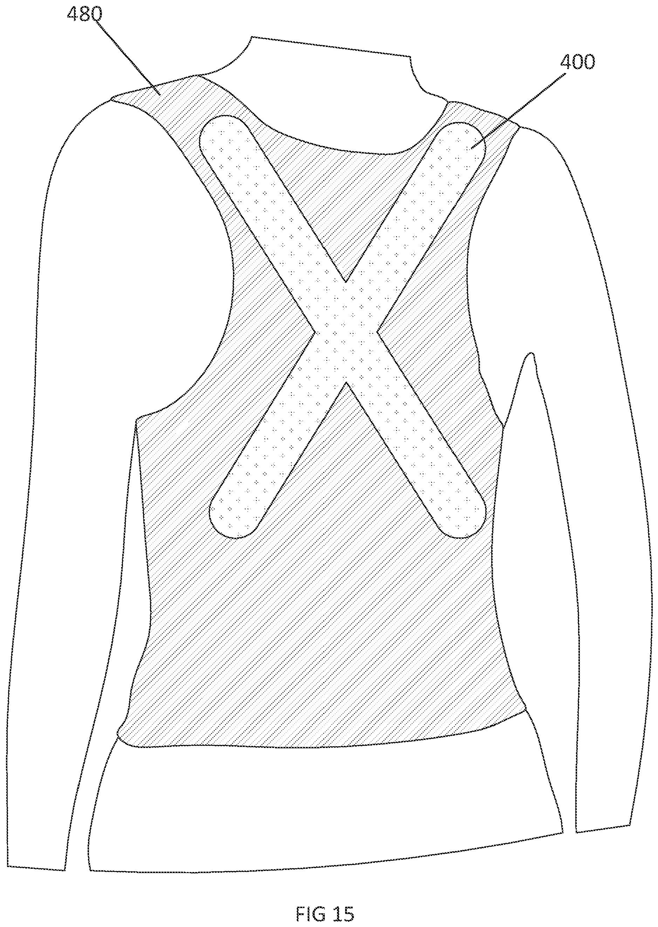

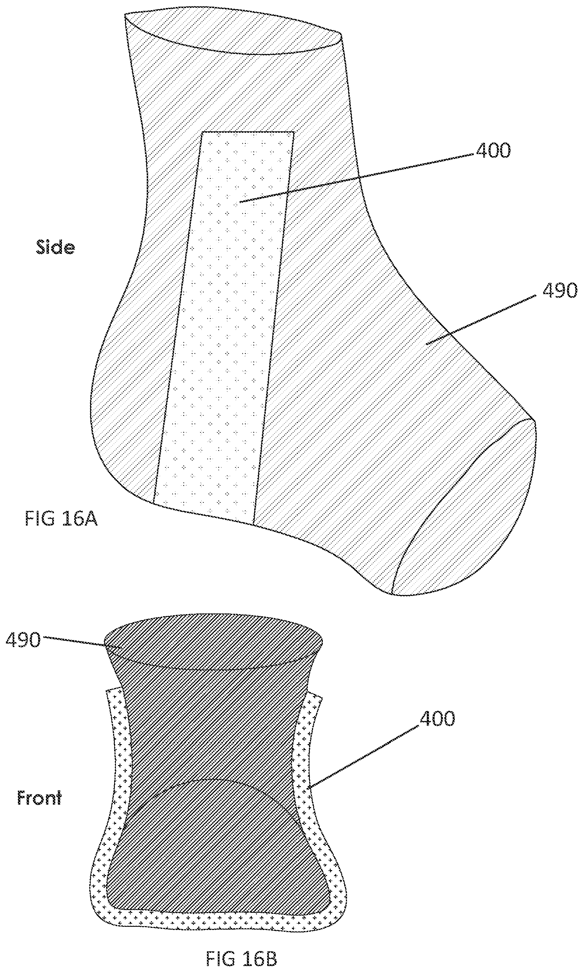

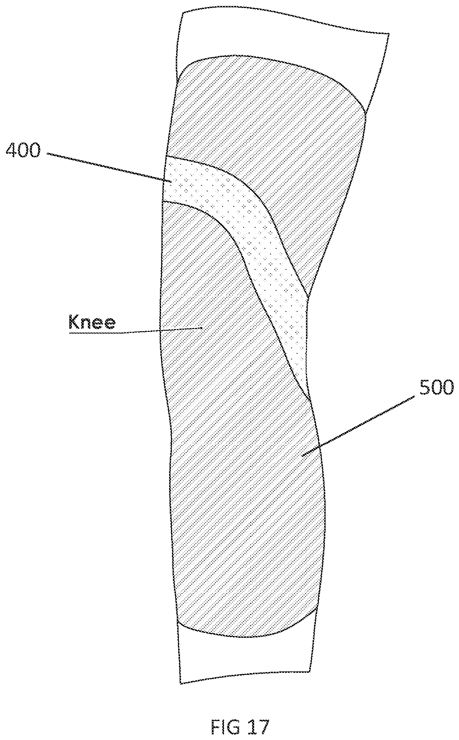

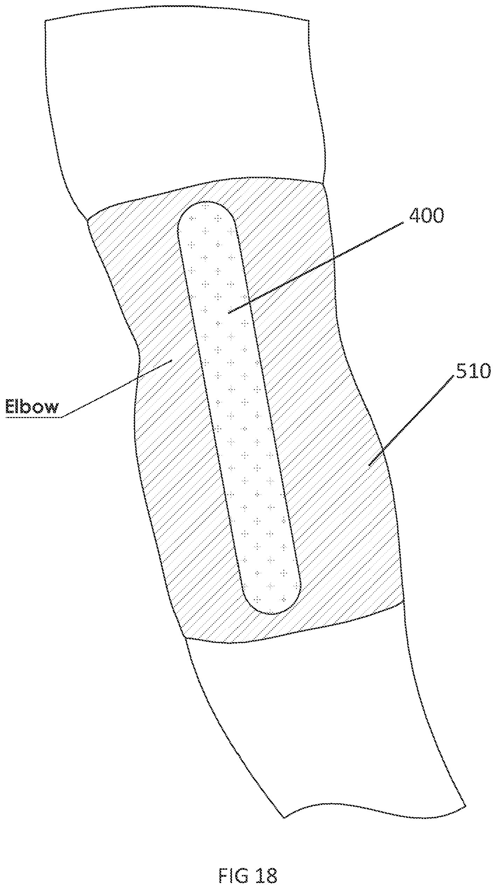

The low extensibility material **400** can also be used with orthotics which is useful to limit joint flexion both as a therapeutic aid in the case of injuries or as a means of injury prevention. As seen in Fig. 14, which does not form part of the claimed invention, the low extensibility material **400** works with a wrist brace **470** and can be adhered externally or internally as described above. Similarly, as shown in Fig. 15, which does not form part of the claimed invention, the low extensibility material **400** can be integrated with a back brace orthotic 480. Likewise, the low extensibility material **400** can be used with an ankle orthotic **490,** as shown in Figs. 16A and 16B, which do not form part of the claimed invention, show thatthe low extensibility material **400** preferably has a thickness to prevent ankle movement. Fig. 17, which does not form part of the claimed invention, depicts the use of the low extensibility material **400** with a knee orthotic **500** and Fig. 18 which does not form part of the claimed invention, depicts the use of the low extensibility material **400** on an elbow orthotic **510.** In all configurations when in use with an orthotic, the low extensibility material **400** can be in a strip or in a specific shape, as determined by an orthotic physician, and be adhered to the fabric **30** either internally or externally.

When the low extensibility material **400** is desired to be used internally, the low extensibility material **400** is first adhered to the fabric **30** on the fabric face **20** using conventional means and then the combination is placed in a mold wherein the gel, silicon, or urethane is molded over, creating a composite. Alternatively, exterior use, which does not form part of the claimed invention, is performed by adhering the low extensibility material **400** onto the outside of the fabric **30** in the desired location.

The present disclosure includes that contained in the appended claims, as well as that of the foregoing description. Although this invention has been described in its preferred form with a certain degree of particularity, it is understood that the present disclosure of the preferred form has been made only by way of example and that numerous changes in the details of construction and the combination and arrangement of parts may be resorted to without departing from the scope of the invention.

## Claims

1. A prosthetic liner (100) comprising:
a closed distal end (80) and an open proximal end (90), the prosthetic liner further comprising an elastomer layer (10) and a fabric layer (30);
said fabric layer being knitted in a tubular shape and comprising a proximal region (60) and a distal region (50);
the elastomer layer comprising an elastomeric gel layer residing on an interior surface (20) of said fabric layer;
wherein said distal region of the fabric layer comprises a knitting style having lower longitudinal elongation than said proximal region; and wherein
said elastomeric gel layer comprises a styrene-based polymer;
**characterized in that** the prosthetic liner (100) further comprises a single piece low extensibility material (400) in between said elastomeric gel layer (10) and said fabric layer (30); and
said single-piece low extensibility material extends up from the closed distal end up to 15,24cm (six inches) from the open proximal end and having a width between 12.7 and 101.6mm (0.5 and 4 inches).

2. The prosthetic liner of claim 1 wherein said knitting style is a stitching selected from the group consisting of weft, warp, stockinette, garter, seam, fagoting, tricot, elongated, plated, slip, dip, or basket stitches.

3. The prosthetic liner of claim 1 further comprising a locking mechanism region (150) below said distal region housing a locking mechanism (130).

4. The prosthetic liner of claim 3 wherein said fabric covering in said locking mechanism region (150) is thicker than said fabric covering in the distal region.

5. The prosthetic liner of claim 1 wherein said fabric covering exhibits greater longitudinal elongation in pressure sensitive areas of a residual limb.

6. The prosthetic liner of claim 1 wherein said fabric covering (30) exhibits lower longitudinal elongation in pressure tolerant areas of a residual limb.

7. The prosthetic liner of claim 1 wherein said distal region (50) can stretch from 0- 30% vertically and 10-200% horizontally.

8. The prosthetic liner of claim 1 wherein the single-piece low extensibility material further comprises a central aperture (420).

## Patentansprüche

1. Prothesenauskleidung (100), umfassend:
ein geschlossenes distales Ende (80) und ein offenes proximales Ende (90), wobei die Prothesenauskleidung ferner eine Elastomerschicht (10) und eine Gewebeschicht (30) umfasst;
wobei die Gewebeschicht in einer röhrenförmigen Form gestrickt ist und einen proximalen Bereich (60) und einen distalen Bereich (50) umfasst;
wobei die Elastomerschicht eine elastomere Gelschicht umfasst, die sich an einer Innenfläche (20) der Gewebeschicht befindet;
wobei der distale Bereich der Gewebeschicht einen Strickstil mit einer geringeren Längsdehnung als der proximale Bereich umfasst, und wobei die elastomere Gelschicht ein Polymer auf Styrolbasis umfasst;
**dadurch gekennzeichnet, dass** die Prothesenauskleidung (100) ferner ein einteiliges Material (400) mit geringer Dehnbarkeit zwischen der elastomeren Gelschicht (10) und der Gewebeschicht (30) umfasst; und
wobei sich das einteilige Material mit geringer Dehnbarkeit von dem geschlossenen distalen Ende bis zu 15,24 cm (sechs Zoll) von dem offenen proximalen Ende erstreckt und eine Breite zwischen 12,7 und 101,6 mm (0,5 und 4 Zoll) aufweist.

2. Prothesenauskleidung nach Anspruch 1, wobei der Strickstil eine Masche ist, die aus der Gruppe ausgewählt ist, die aus Schuss-, Kett-, Baumwollnetz-, Strumpfband-, Naht-, Fagotting-, Trikot-, länglichen, plattierten, Hebe-, Tauch- oder Korbmaschen besteht.

3. Prothesenauskleidung nach Anspruch 1, ferner umfassend einen Arretiermechanismusbereich (150) unter dem distalen Bereich, der einen Arretiermechanismus (130) aufnimmt.

4. Prothesenauskleidung nach Anspruch 3, wobei die Gewebeabdeckung in dem Arretiermechanismusbereich (150) dicker ist als die Gewebeabdeckung in dem distalen Bereich.

5. Prothesenauskleidung nach Anspruch 1, wobei die Gewebeabdeckung eine größere Längsdehnung in druckempfindlichen Bereichen eines Restglieds aufweist.

6. Prothesenauskleidung nach Anspruch 1, wobei die Gewebeabdeckung (30) eine geringere Längsdehnung in drucktoleranten Bereichen eines Restglieds aufweist.

7. Prothesenauskleidung nach Anspruch 1, wobei sich der distale Bereich (50) von 0 bis 30 % vertikal und 10 bis 200 % horizontal erstrecken kann.

8. Prothesenauskleidung nach Anspruch 1, wobei das einteilige Material mit geringer Dehnbarkeit ferner eine zentrale Öffnung (420) umfasst.

## Revendications

1. Revêtement de prothèse (100) comprenant :
une extrémité distale fermée (80) et une extrémité proximale ouverte (90), le revêtement de prothèse comprenant en outre une couche d'élastomère (10) et une couche de tissu (30) ;
ladite couche de tissu étant tricotée dans une forme tubulaire et comprenant une zone proximale (60) et une zone distale (50) ;
ladite couche d'élastomère comprenant une couche de gel élastomère résidant sur une surface interne (20) de ladite couche de tissu ;
ladite zone distale de la couche de tissu comprenant un style de tricotage présentant un allongement longitudinal inférieur à celui de ladite zone proximale, et ladite couche de gel élastomère comprenant un polymère à base de styrène ;
**caractérisé en ce que** le revêtement de prothèse (100) comprend en outre un matériau à faible extensibilité (400) d'une seule pièce entre ladite couche de gel élastomère (10) et ladite couche de tissu (30) ; et
ledit matériau à faible extensibilité d'une seule pièce s'étendant depuis l'extrémité distale fermée jusqu'à 15,24 cm (six pouces) de l'extrémité proximale ouverte et présentant une largeur comprise entre 12,7 et 101,6 mm (0,5 et 4 pouces).

2. Revêtement de prothèse selon la revendication 1, ledit style de tricotage étant une couture choisie dans le groupe constitué par les mailles de trame, de chaîne, de jersey, à points mousses, à points de couture, à points de chausson, de tricot, allongées, plaquées, coulées, vers le bas ou à points de damier.

3. Revêtement de prothèse selon la revendication 1, comprenant en outre une zone de mécanisme de verrouillage (150) en dessous de ladite zone distale logeant un mécanisme de verrouillage (130).

4. Revêtement de prothèse selon la revendication 3, ladite couverture de tissu dans ladite zone de mécanisme de verrouillage (150) étant plus épaisse que ladite couverture de tissu dans la zone distale.

5. Revêtement de prothèse selon la revendication 1, ladite couverture de tissu présentant un allongement longitudinal plus important dans les régions sensibles à la pression d'un membre résiduel.

6. Revêtement de prothèse selon la revendication 1, ladite couverture de tissu (30) présentant un allongement longitudinal inférieur dans les régions tolérantes à la pression d'un membre résiduel.

7. Revêtement de prothèse selon la revendication 1, ladite zone distale (50) pouvant s'étirer de 0 à 30 % verticalement et de 10 à 200 % horizontalement.

8. Revêtement de prothèse selon la revendication 1, ledit matériau à faible extensibilité d'une seule pièce comprenant en outre une ouverture centrale (420).
